# EUROPEAN PATENT SPECIFICATION

(11) **EP 4 362 771 B1**
(45) Date of publication and mention of the grant of the patent: **05.08.2026**
(21) Application number: 22738625.7
(22) Date of filing: 28.06.2022
(51) Int. Cl.: A61B 5/00

(54) **DRY BIOPOTENTIAL ELECTRODE WITH PERFORATIONS**
TROCKENE BIOPOTENTIALELEKTRODE MIT PERFORATIONEN
ÉLECTRODE BIOPOTENTIELLE SÈCHE À PERFORATIONS

(30) Priority: 30.06.2021 EP 21182862
(43) Date of publication of application: 08.05.2024
(73) Proprietor: Nanoleq AG, 8153 Rümlang (CH)
(72) Inventor: MARTINEZ, Vincent, 8049 Zurich (CH); STAUFFER, Flurin, 8050 Zurich (CH)
(74) Representative: Vossius & Partner Patentanwälte Rechtsanwälte mbB
(86) International application number: PCT/EP2022/067680
(87) International publication number: WO 2023/275019

(56) References cited:
- WO-A1-2012/066056
- WO-A1-2018/004614
- CN-A- 106 859 637

## Description

The present invention relates to a textile electrode, a textile product comprising a textile electrode and to a method of manufacturing a textile electrode.

In recent years, health-monitoring textiles have become more and more important. Such health-monitoring textiles require certain textile sensors such as textile-integrated electrodes. To measure a high-quality biosignal with a textile-integrated electrode, a low and stable electrode-skin impedance is required. However, the skin conditions of a user may vary during use from extremely dry to soaked, e.g., due to sweat. This leads to a high variety of impedance levels spanning from e.g., 20 kΩ to more than 2 MΩ. Such high varieties can occur due to different skin types and skin treatments among different people on the one hand, and external influences such as air temperatures and air humidity on the other hand. For example, during low temperatures and low air humidity in winter the skin tends to be driest, causing a higher skin impedance when interfaced with dry electrodes. Moreover, movements at the electrode-skin interface can create impeding motion artifacts. The higher the impedance and the bigger the impedance changes, the bigger these artifacts. In addition, when the textile-integrated electrode is integrated in garments there is only a limited electrode-skin pressure applicable.

It is thus an object of the present invention to provide an improved textile electrode which allows measuring a high-quality biosignal by maintaining a low and stable electrode-skin impedance. This object is achieved with a textile electrode according to claim 1 (as well as the corresponding textile product and method of manufacturing the textile electrode). The dependent claims relate to preferred embodiments.

Accordingly, the present invention provides a textile electrode comprising a backing and a laminar electrode. The backing comprises, and preferably consists of, a textile material. The laminar electrode has first and second surfaces, the first surface being arranged and configured to be in direct contact with skin of a person during use and the second surface being (directly or indirectly) attached to the textile backing. The laminar electrode is electrically conductive and comprises through holes extending from the first surface to the second surface of the laminar electrode. The laminar electrode, at areas without any through holes, has a higher resistance to water penetration than the textile backing. At areas without any through holes, the laminar electrode has a resistance to water penetration that is preferably greater than 10 mBar.

The combination of the textile backing and the through holes in the laminar electrode provides two technical advantages. First and foremost, the through holes allow for electrode-skin wetting from outside of a textile product comprising the textile electrode when it is already being worn. This substantially reduces the electrode-skin impedance and the signal quality of the recorded signal during the first minutes of wear and improves the user experience as it is more convenient to wet the electrodes after the textile product has been put on by the user. When wetting the textile backing from outside, water can penetrate through the water-permeable textile backing as well as through the through holes of the laminar electrode and thus wet the skin of the user underneath the laminar electrode. In addition, wetting of an electrode may help to establish a low enough electrode-skin impedance faster than when relying on wetting of the electrode by sweat, i.e., the wetting may help to reduce the electrode settling time. Thus, it may be understood that the through holes are not sealed with and/or occluded by any material. In other words, the through holes are configured and/or capable of transporting water.

A second advantage of the textile electrode of the present invention will be apparent during long term use when sweat generated by the user underneath the electrode accumulates in the through holes and is drained by the textile backing. This can avoid skin irritations and lifting of the electrode from the skin. Accordingly, the adhesion of the electrodes to the skin is improved which ensures a stable electrode-skin interface and, hence, improved signal quality during movements of the user.

So far, no electrodes available in the prior art (a review of which may be found, e.g., in Hao Wu et al.: "Materials, Devices, and Systems of On-Skin Electrodes for Electrophysiological Monitoring and Human-Machine Interfaces", Advanced Science, vol. 8, 2, January 20, 2021) provide the above-mentioned benefits. For example, conventional dry electrodes (described, for example, in US 8,355,770 B2) are not wettable from outside, do not provide sweat drainage and can, thus, induce sweating between skin and electrode. Microporous, hydrophobic electrodes (described, for example, in CN 106859637) are also not wettable from the outside and do not drain any liquid sweat. Moreover, the contact surface area between the electrode and the skin is substantially reduced for such microporous electrodes. While electrodes that are microporous and hydrophilic (described, for example, in US 2020/0046246 A1) do drain sweat, the contact surface area between the electrode and the skin is substantially reduced which causes a worse contact impedance, especially in dry conditions. In general, decreasing the contact surface area between the electrode and the skin causes an increase in the electrode-skin impedance in dry conditions and lowers the skin adhesion, both potentially negatively impacting the biopotential signal quality, especially during movements of the user. Other relevant prior art is disclosed in WO2012/066056 and WO2018/004614.

The resistance to water penetration may be measured according to ISO 811:2018 using a test speed of 60 cm water column per minute. Ideally, the resistance to water penetration should be measured for a test specimen having an area of 100 cm² as specified in ISO 8911:2018. However, as the resistance to water penetration of the electrode material should not take the effect of any through hole into account, the measurement may be performed with a circular section of the electrode having an area of 1 cm² which does not comprise a single through hole. Should such an area not be available, the through holes may be sealed with silicone sealant for silicone electrodes (such as silicone sealant Dow corning 734) or with respective sealants appropriate for the electrode materials (e.g. rubber or polyurethane sealants) before performing the measurement. The resistance to water penetration of the laminar electrode shall be greater than 10 mBar, preferably greater than 25 mBar and more preferably greater than 50 mBar.

By providing the electrode material, at areas without any through holes, with a resistance to water penetration that is greater than 10 mBar, capillary forces occurring in the through holes may be amplified. In this way, the transport of water to the person's skin may be improved.

Preferably, at least 50%, more preferably at least 75%, even more preferably at least 90%, even more preferably 100% of the first surface is arranged and configured to be in direct contact with skin of a person during use.

Preferably, the first surface arranged and configured to be in direct contact with the skin of a person during use is a continuous and/or smooth surface. In other words, it may be understood that the first surface does not comprise any steps, bumps, protrusions or the like. More preferably, the first surface is flat.

Preferably, the first surface of the laminar electrode, at areas without any through holes, has a higher water contact angle than the outer surface of the textile backing. Preferably, the water contact angle of the outer surface of the textile backing is smaller than 90°, more preferably smaller than 60° and most preferably smaller than 30°. It is particularly preferable that water is fully wetting the textile backing.

The water contact angle may be measured according to ASTMD 5725-99, wherein the contact angle is measured 10 seconds after initial contact between the water droplet and the respective surface.

The textile backing is water permeable. Preferably, the volume of water that penetrates through the backing within 10 minutes amounts to at least 100 ml, more preferably to at least 250 ml and most preferably to at least 500 ml when exposed to water drops that amount to 800 ml of water in total. The water permeability may be measured according to ISO 9865:1991.

At areas without any through holes, the laminar electrode preferably has a water vapor transmission rate lower than 10,000 g/m²/d, preferably lower than 5,000 g/m²/d, most preferably lower than 2,000 g/m²/d. The water vapor transmission rate may be measured according to JIS L 1099-B1.

The through holes may generally have any shape. However, cylindrical through holes with a circular cross-section are preferred. The cross-sectional area of each through hole is preferably greater than 0.4 mm², more preferably greater than 1 mm², and most preferably greater than 2 mm². The cross-sectional area of each through hole is preferably smaller than 50 mm², more preferably smaller than 30mm² and most preferably smaller than 10 mm². If the cross-section varies along the thickness of the laminar electrode, these dimensions preferably apply to the cross-sectional area of the smallest cross-section.

The total cross-sectional area of all through holes preferably amounts to at least 3%, more preferably at least 5% and most preferably at least 7% of the area of the first surface of the electrode. Preferably, the total cross-sectional area of all through holes amounts to at most 30%, more preferably at most 20% and most preferably at most 10% of the area of the first surface of the electrode.

The cross-section of the through holes has a maximum dimension and the laminar electrode has a thickness. Preferably, the ratio of the maximum dimension to the thickness is greater than 1, more preferably greater than 2, even more preferably greater than 3 and most preferably greater than 4.

The through holes are preferably provided in a homogenous, more preferably a regular, pattern over the laminar electrode or over at least an area of the laminar electrode.

Preferably, the textile material of the backing has directional water transport properties with a preferred direction going from a surface of the textile backing facing away from the laminar electrode towards the surface of the textile backing attached to the second surface of the laminar electrode.

The electrical conductivity of the laminar electrode is preferably greater than 1 S/m, more preferably greater than 10 S/m and most preferably greater than 100 S/m.

The laminar electrode may consist of a single layer. It is, however, preferred that the laminar electrode comprises two or more layers. For example, the laminar electrode may comprise a conductive fabric attached to the textile backing and an additional conductive layer arranged to be in direct contact with skin of a person.

The conductive fabric preferably has an electrical conductivity of at least 10² S/m, more preferably at least 10³ S/m and most preferably at least 10⁴ S/m. Furthermore, the conductive fabric preferably has a sheet resistance of less than 10 Ω/sq ("ohms per square"), more preferably less than 5 Ω/sq, most preferably less than 2 Ω/sq.

Preferably, the conductive fabric has a greater electrical conductivity than the additional conductive layer as the conductive fabric may be used to, e.g., provide electrical contact to an electrical connector being in electrical contact with the laminar electrode. The additional conductive layer preferably has an electrical conductivity of at least 0.1 S/m, more preferably at least 1 S/m and most preferably at least 10 S/m.

Preferably, the conductive layer is adhesive to skin. The adhesion to the skin is preferably higher than 0.1 N/cm², more preferably higher than 0.5 N/cm², most preferably higher than 1 N/cm². For this purpose, the conductive layer may, e.g., be silicone-based and may comprise carbon and/or silver. The conductive fabric may, e.g., be a fabric comprising silver. A particularly preferred conductive fabric is a fabric based on nylon coated with silver.

Preferably, the laminar electrode is attached to the textile backing by means of an adhesive layer. The adhesive layer may comprise one or a combination of the following materials: polyurethane, silicone.

Preferably, the conductive fabric and the conductive layer are attached to each other by means of an adhesive layer. The adhesive layer is preferably also conductive. The conductive layer may also be laminated or printed onto the conductive fabric.

The adhesive of the adhesive layer may penetrate at least a portion of the adjacent layers, i.e. at least a portion of the conductive fabric and/or the textile backing.

The adhesive layer between the laminar electrode and the textile backing comprises perforations. The perforations preferably have a maximum cross-section between 0.2 mm² and 20 mm², preferably between 0.4 mm² and 8 mm², and most preferably between 0.7 mm² and 3.5 mm².

The textile electrode may further comprise a non-conducting ring surrounding the laminar electrode. The non-conducting ring may comprise various layers such as a textile layer, an adhesive layer and/or a layer based on, e.g., silicone or polyurethane. Preferably, the electrical conductivity of the non-conductive ring is smaller than 10⁻⁴ S/m, more preferably smaller than 10⁻⁷ S/m, and most preferably smaller than 10⁻¹⁰ S/m. Preferably, the non-conducting ring has anti-slip properties on human skin or even more preferably adhesion properties to the skin.

Preferably, the non-conducting ring is separated from the laminar electrode by means of a recess. This recess may have a similar effect as the through holes, i.e., the recess may also allow for wetting of the skin under the laminar electrode 3. It is thus preferred that the recess has a width between 0.3 mm and 4 mm, preferably between 0.5 mm and 2 mm, more preferably between 0.8 mm and 1.2 mm.

In order to collect the signals generated at the laminar electrode, the textile electrode preferably further comprises an electrical connector, which may be attached to an electrical wire. Preferably, one end of the electrical connector or the entire electrical connector is sandwiched between the backing and the laminar electrode so as to establish an electrical connection between the laminar electrode and the electrical connector. Preferably, the laminar electrode is attached to the backing by means of an adhesive layer, wherein the one end of the electrical connector is sandwiched between the backing of the adhesive layer of the laminar electrode. Preferably, the adhesive layer comprises perforations in an area sandwiched between the laminar electrode and the one end of the electrical connector so as to establish an electrical connection between the laminar electrode and the electrical connector.

The present invention further relates to a textile product comprising the textile electrode as described above. Preferably, the textile material of the backing of the electrode is an integral part of the textile of the textile product. In other words, the second surface of the laminar electrode is preferably directly attached to a textile layer of the textile product. The textile product may be any product that might benefit from an integrated electrode such as a T-shirt, a bra, a sport bra, undergarment, undershirt, a base layer, a sweatshirt, a shirt, a dress, underwear, a jacket, vest, a head band, a cap, pants, socks, swim wear, a swim suit, a diving suit, a bikini, compression garments, sport and medical bandages, a facemask, protective equipment, sports equipment, shoes, a helmet, headphones, gloves, sleeves, bracelets, watches, belts, a corset, a bodice, a one piece, a scarf.

The present invention further relates to a method of manufacturing the textile electrode as described above. The method comprises providing an electrically conductive laminar electrode with first and second surfaces, the first surface being arranged to be in direct contact with skin of a person, wherein the electrode material has a resistance to water penetration that is greater than 10 mBar. The method further comprises generating through holes in the laminar electrode extending from the first surface to the second surface of the laminar electrode. The method further comprises providing a textile backing, wherein the laminar electrode has a higher resistance to water penetration than the textile backing. Finally, the second surface of the laminar electrode is attached to the textile backing.

Of course, all features discussed above with regard to the textile electrode of the present invention may be employed analogously in the context of the method according to the present invention. This is, in particular, true for the material properties discussed above for the various layers of the textile electrode. However, in the context of the method the material properties may be measured for the materials before generating the through holes and before attaching the layers to each other.

Accordingly, it is preferred that the textile backing is water permeable, wherein the volume of water that penetrates through the textile backing (without the other components of the electrode being attached thereto) within 10 minutes preferably amounts to at least 100 ml, more preferably to at least 250 ml and most preferably to at least 500 ml when exposed to water drops that amount to 800 ml of water in total. It is further preferred that the laminar electrode, before generating the through holes, has a water vapor transmission rate lower than 10,000 g/m²/d, preferably lower than 5,000 g/m²/d, most preferably lower than 2,000 g/m²/d.

Preferably, the first surface of the laminar electrode has a higher water contact angle than the surface of the textile backing which will not be attached to the laminar electrode.

Preferably, the step of providing an electrically conductive laminar electrode comprises providing a perforated adhesive layer, bonding the perforated adhesive layer to a conductive fabric and coating the conductive fabric with a, preferably adhesive, conductive layer.

As discussed above, the present invention allows the user to get liquid water through the textile backing onto the skin and therefore increases the skin humidity, which allows for improved electrode-skin contact and better biosignal recording quality. When the user wets the outer textile layer, it takes up the water. The textile backing is preferably selected so that it releases excessive water easily through the holes in the laminar electrode and this water remains in the holes and wets the skin. The combination of the above-discussed features allow the liquid water to prefer to stay on the skin rather than in the textile backing to improve the liquid transfer.

If water is applied onto the textile backing under pressure, the wet textile can bend down allowing the liquid to touch the skin. If the pressure is released, the textile withdraws. If the liquid prefers to stay on the textile, the liquid transfer is limited. The more hydrophobic the surface of the textile and the closer to the textile is approached to the skin that is hydrophobic, the easier water droplets stay on the skin.

Preferred embodiments of the present invention will now be further elucidated with reference to the Figures which show:
- Figure 1: a bottom view of a preferred embodiment of a textile electrode according to the present invention;
- Figure 1A: a cross-section through the textile electrode of Figure 1 along line A;
- Figure 1B: a cross-section through the textile electrode of Figure 1 along line B;
- Figure 2A: a cross-section through a textile electrode according to another preferred embodiment of the present invention along line A of Figure 1;
- Figure 2B: a cross-section through a textile electrode according to the embodiment of Figure 2A along line B of Figure 1;
- Figure 3: a bottom view of a preferred embodiment of a textile electrode according to the present invention;
- Figure 3A: a cross-section through the textile electrode of Figure 3 along line A;
- Figure 3B: a cross-section through the textile electrode of Figure 3 along line B;
- Figure 4A: a cross-section through a textile electrode according to another preferred embodiment of the present invention along line A of Figure 3;
- Figure 4B: a cross-section through a textile electrode according to the embodiment of Figure 4A along line B of Figure 3;
- Figure 5: a bottom view of a laminar electrode of a textile electrode according to a preferred embodiment of the present invention;
- Figure 5A: a top view of the laminar electrode of Figure 5;
- Figure 5B: a cross-section through the laminar electrode of Figure 5 along line B;
- Figure 5C: a cross-section through the laminar electrode of Figure 5 along line C;
- Figure 6: schematically a method of manufacturing a textile electrode with an electrical contact according a to preferred embodiment of the present invention;
- Figure 7: the assembled textile electrode with the electrical contact according to the embodiment of Figure 6;
- Figure 8: the result of a measurement of electrode-skin impedance;
- Figure 9: the result of a measurement of electrode-skin impedance with repetitive external wetting;
- Figure 10: the result of a control measurement of electrode-skin impedance over time;
   and
- Figure 11: a photograph of an exemplary textile product after intensive exercising.

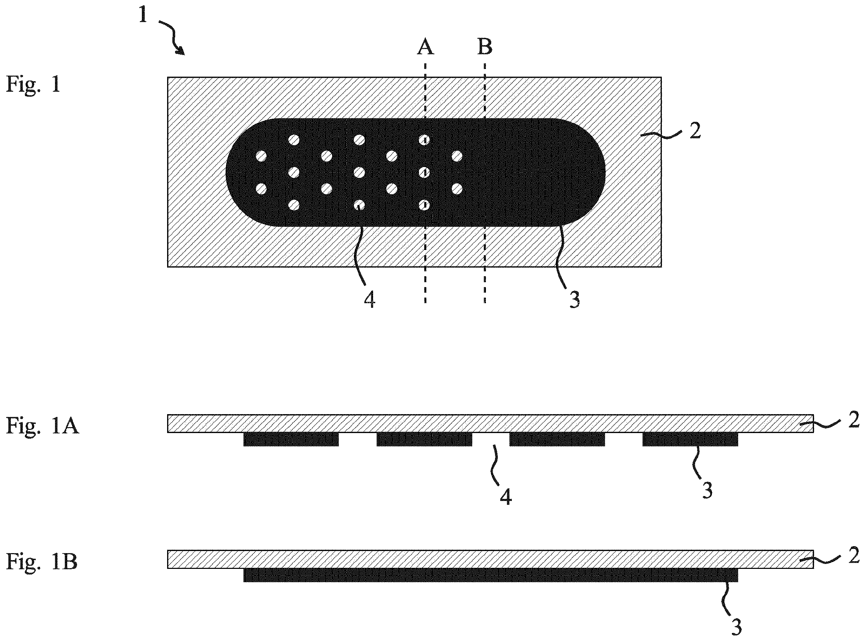

Figure 1 shows a bottom view of a textile electrode 1 according to a preferred embodiment of the present invention. The textile electrode 1 comprises a textile backing 2 and a laminar electrode 3 with a first surface (visible in Figure 1) and a second surface attached to the textile backing 2. The first surface of the laminar electrode 3 is supposed to be arranged in direct contact with the skin of a person. The laminar electrode 3 is electrically conductive and comprises several through holes for extending from the first surface to the second surface of the laminar electrode (see the cross-section in Figure 1A). As mentioned above, the laminar electrode of the present invention comprises a material which, at areas without any through holes 4, has a higher resistance to water penetration and a higher water contact angle than the material of the textile backing 2. At areas without any through holes 4, the laminar electrode 3 further has a resistance to water penetration that is greater than 10 mBar.

In the embodiment shown in Figure 1, the textile backing 2 has a rectangular shape and the laminar electrode 3 has an oval shape. It will, however, be apparent to the skilled person that other shapes may be employed with different relative sizes as well. For example, the laminar electrode 3 may also be circular, elliptical, quadratic or rectangular in shape or may have any other irregular or regular shape suitable for an electrode. The textile backing 2 will, in many cases, be an integral part of a larger textile product such as a T-shirt. Accordingly, the actual shape of the textile backing 2 is of no relevance for the present invention.

The through holes 4 are shown to be cylindrical having a circular cross-section in Figures 1 and 1A. However, again other shapes are envisaged as well for the through holes 4 and may also be circular, elliptical, quadratic or rectangular in shape or may have any other irregular or regular shape. The through holes 4 should be arranged in a somewhat homogeneous or even regular pattern over the laminar electrode 3. In Figure 1, a hexagonal pattern has been chosen to arrange the through holes 4 equidistantly.

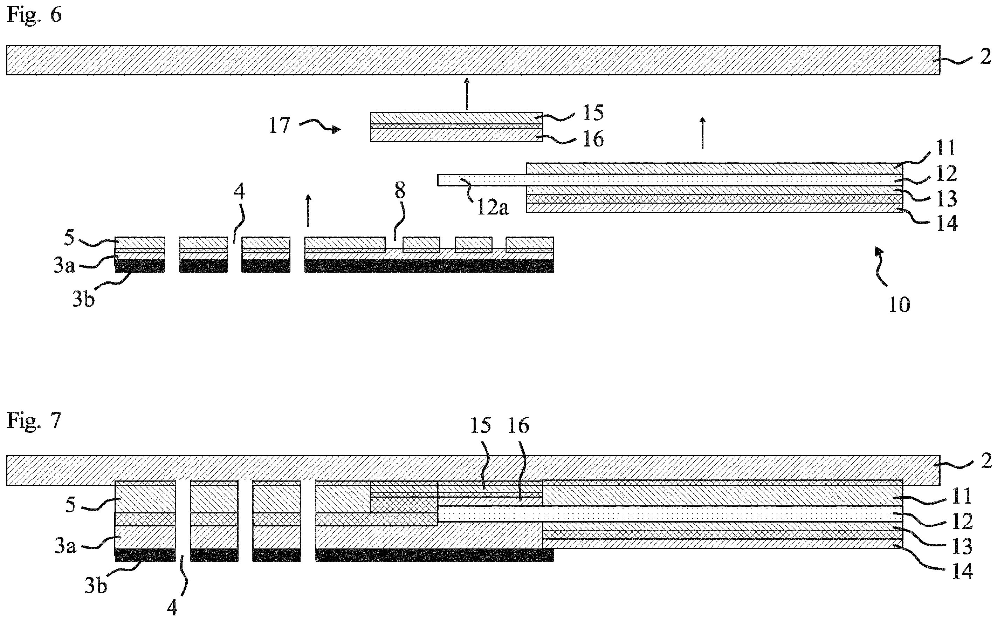

While such an arrangement is preferable in terms of achieving a homogeneous effect, other arrangements of the through holes 4 are also possible. As shown in Figure 1, the through holes 4 need not be spread out over the entire surface of the laminar electrode 3. Rather, a certain portion of the laminar electrode 3 may also be free of such through holes 4. The reasons therefor will be evident when discussing Figures 6 and 7 below.

The textile backing may comprise any textile material known for textile products and may comprise one or a combination of the following materials: elastane, polyamide, nylon, polyester, polyethylene, polyurethane, polypropylene, viscose. Preferably, the weight of the textile backing is less than 600 g/m², more preferably less than 400 g/m², most preferably less than 250 g/m² and/or the density of the textile backing is smaller than 1.5 g/cm³, more preferably smaller than 1.1 g/cm³, most preferably smaller than 0.8 g/cm³.

The material of the laminar electrode is optimized to adapt to the skin of a user. Preferably, the material of the laminar electrode, at least at the first surface, is somewhat adhesive to human skin. Particularly preferable laminar electrodes are based on silicone and comprise carbon and/or silver in order to become conductive.

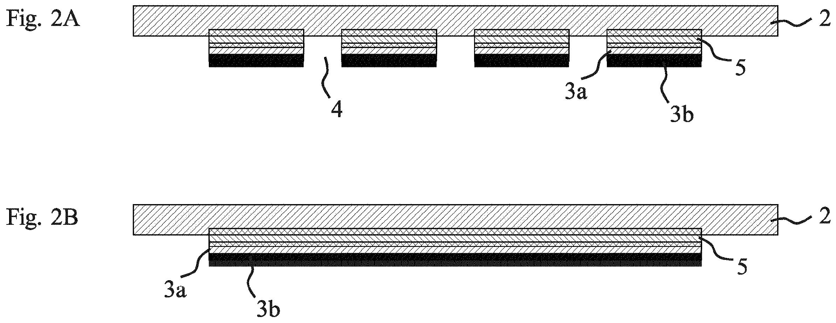

The laminar electrode 3 is attached to the textile backing 2 by, e.g., a suitable adhesive material. Moreover, the laminar electrode 3 may comprise several layers as shown in the embodiment of Figures 2A and 2B. In this embodiment, the laminar electrode 3 comprises a conductive fabric 3a and a further conductive layer 3b. The conductive fabric is preferably more conductive than the additional conductive layer and may, e.g., be a fabric comprising silver. Said conductive fabric 3a may be directly covered with the additional conductive layer 3b. For example, a carbon-silicone-based conductive layer 3b may be printed in liquid state onto the conductive fabric 3a and subsequently vulcanized. Alternatively, the conductive layer 3b may be directly laminated onto the conductive fabric 3a.

The entire laminar electrode 3 is preferably attached to the textile backing 2 by means of an adhesive layer 5. Suitable adhesive materials are the following: polyurethanes, silicones.

Since both the laminar electrode and the adhesive layer 5 may not be sufficiently water-permeable, it is preferred that the through holes 4 extend not only through the entire laminar electrode (i.e. from the first surface to the second surface), but also through the adhesive layer 5. In other words, it is preferred that the through holes 4 extend from the first surface of the laminar electrode all the way to the surface of the textile backing 2 as shown in Figure 2A.

If the textile backing 2 is, for example, part of a T-shirt, said textile backing 2 may be wetted while the T-shirt is being worn by a user. The water then penetrates the textile backing 2, enters into the trough-holes 4 and thus wets the skin of the user being in contact with the first surface of the laminar electrode 3. Various experiments of the inventors (some of which are discussed further below) have shown that the impedance between the skin and the laminar electrode 3 is substantially reduced after wetting which improves the signal quality. The change of impedance may be caused by water simply wetting the bottom and, in particular, the edge of the hole, where the skin contacts the electrode. Moreover, the water may creep into the gap between the skin and the electrode adjacent to the hole. The more water one applies, the more the skin underneath the laminar electrodes is getting moist/wetted, eventually even reaching similar values as achieved by fully wetting the skin before applying the laminar electrode to the skin.

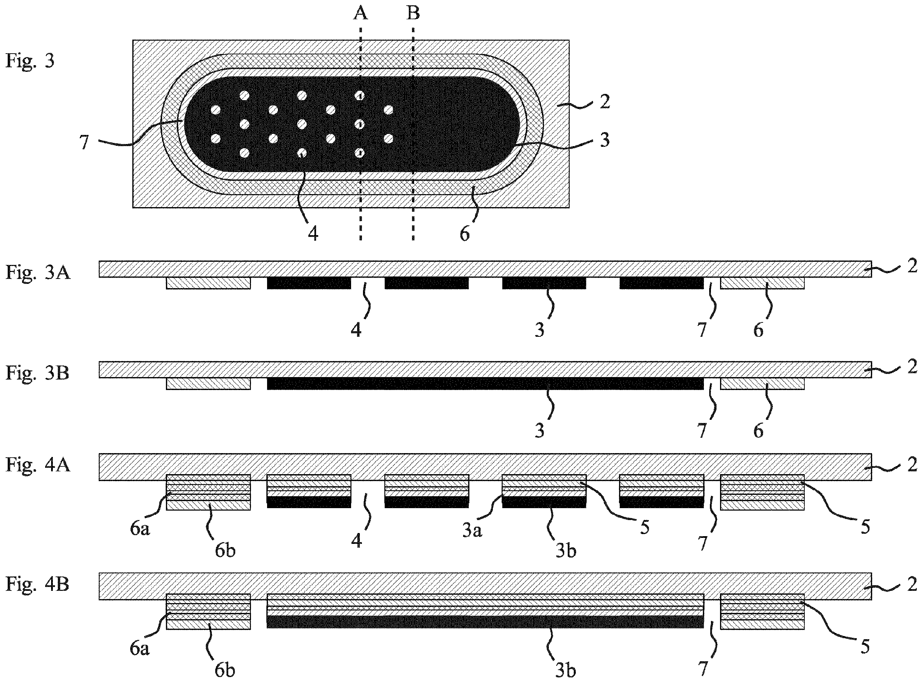

Figure 3 shows a bottom view of another preferred embodiment of a textile electrode according to the present invention. Again, various cross-sections are shown in Figures 3A and 3B (with the laminar electrode 3 consisting of a single layer) and Figures 4A and 4B (with a multi-layered laminar electrode 3). In the embodiments shown in Figures 3-4B, the textile electrode further comprises a non-conducting ring 6 surrounding the laminar electrode 3. Similar to the laminar electrode 3, the non-conducting ring 6 may consist of a single layer such as non-conducting elastomers such silicone, polyurethane, rubber, or a textile fabric. Alternatively, the non-conducting ring 6 may comprise a non-conducting textile 6a and a non-conducting silicone layer 6b directly attached to each other by printing and subsequent vulcanizing, lamination or by means of an additional layer of adhesive. The entire non-conductive ring 6 may be attached to the textile backing 2 by means of a further layer of adhesive 5. In the embodiments shown in Figures 3-4B, the non-conductive ring 6 is separated from the laminar electrode 3 by means of a recess 7. This recess may have a similar effect as the through holes, i.e. the recess may also allow for wetting of the skin under the laminar electrode 3. It is thus preferred that the recess has a width between 0.3 mm and 4 mm, preferably between 0.5 mm and 2 mm, more preferably between 0.8 mm and 1.2 mm. The non-conductive ring 6 may, however, also be in direct contact with the laminar electrode 3.

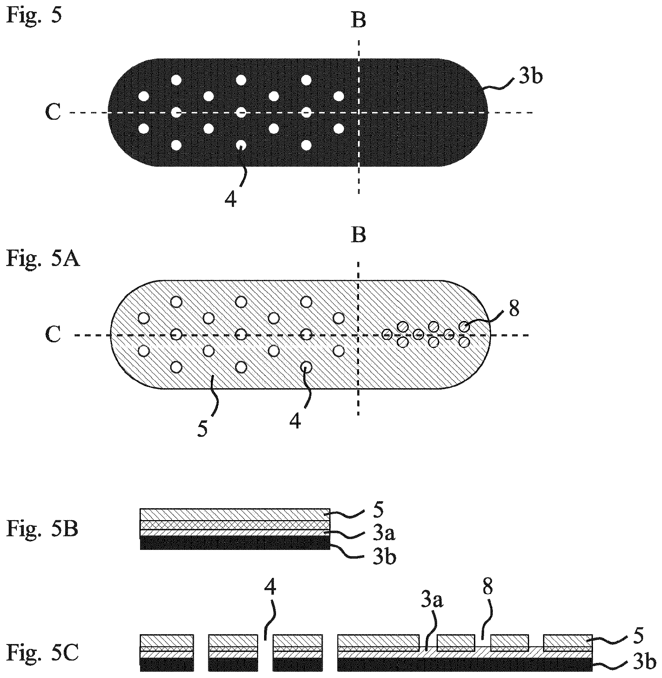

As discussed in more detail below, the textile electrode of the present invention may comprise an electrical connector, wherein one end of the electrical connector is sandwiched between the textile backing 2 and the laminar electrode 3 so as to establish an electrical connection between the laminar electrode 3 and the electrical connector. Since the layer of adhesive 5, with which the laminar electrode 3 is attached to the textile backing 2, will typically be non-conductive, it is preferred to provide several perforations in an area of the adhesive layer 5 sandwiched between the laminar electrode 3 and the one end of the electrical connector so as to establish an electrical connection between the laminar electrode 3 and the electrical connector. A preferred embodiment of a laminar electrode 3 with such perforations 8 being present in the adhesive layer 5 is shown in Figures 5 to 5C. As is evident from Figures 5 and 5A, the laminar electrode 3 may be divided into two sections (here: left and right), wherein several through holes 4 are provided in one section (here: left), whereas the perforations 8 in the adhesive layer 5 are provided in the other section (here: right). Contrary to the through holes 4, which are preferably distributed homogeneously, the perforations 8 may be concentrated in a small stripe or other restricted area of the laminar electrode 3, which will, later on, come into contact with the electrical connector.

Figure 7 shows a cross-section through a preferred embodiment of a textile electrode according to the present invention with an electrical connector. Figure 6 schematically shows how the various components of the textile electrode shown in Figure 7 are assembled.

When manufacturing the textile electrode of the present invention, the electrical conductive laminar electrode 3 with first and second surfaces is first provided or manufactured in the preferred example shown in Figure 6, the adhesive layer 5 is first perforated with perforations 8. The perforated adhesive layer 5 is then bonded to the conductive fabric 3a, which is afterwards (or previously) coated with the additional conductive layer 3b.

Furthermore, an electrical contact patch 10 is prepared, which comprises an electrical contact or wire 12, adhesive layers 11 and 13 and the layer 14 of textile fabric or another suitable cover material. One end 12a of the electrical connector 12 protrudes from the electrical connector patch 10 so as to allow for establishing an electrical connection between the electrical connector 12 and the laminar electrode 3.

A further connection patch 17 comprises a layer of adhesive 15 and a layer of electrically conductive glue or fabric 16. In order to manufacture the textile electrode, said connection patch 17 is laminated onto the textile backing 2. Subsequently, the electrical connector patch 10 is laminated onto the textile backing 2 and the connection patch 17. Finally, the laminar electrode 3 is laminated onto the textile backing 2, the connection patch 17 and the electrical connector patch 10 so as to achieve the assembled product as shown in Figure 7.

In the final product, electrical signals collected by the additional conductive layer 3b of the laminar electrode 3 are transmitted through the conductive fabric 3a of the laminar electrode 3 and through the perforations 8 of the adhesive layer 5 into the end 12a of the electrical connector 12. Wetting may take place on the left side of the laminar electrode 3 through the through holes 4 and through the recess 7 (in case there is a non-conductive ring). As is evident from Figure 7, no through holes 4 are required on the right side of the laminar electrode 3, because wetting would be anyway blocked by the additional layers of the connection patch 17.

In order to show the beneficial effect of the textile electrode of the present invention, the following measurement has been performed.

The exemplary textile electrode used for the measurement had a composition as shown in Figure 4A and 4B and consisted of an adhesive carbon silicone layer 3b, conductive fabric (nylon, silver) 3a, a polyurethane adhesive layer 5 and a textile backing (71% polyester, 27% polyamide and 2% elastane) 2. The electrode had a length of 70 mm and a width of 20 mm with 15 through holes with a diameter of 3 mm and a non-conducting ring 6 made of an adhesive silicone 6b, a textile layer (72% polyamide, 28% elastane) 6a and a polyurethane adhesive layer 5 with a recess 7 of approximately 0.8 mm width. Two exemplary textile electrodes were incorporated into a T-Shirt made of 71% polyester, 27% polyamide and 2% elastane that acts as the textile backing layer 2. The electrodes were positioned below the chest muscle on the chest.

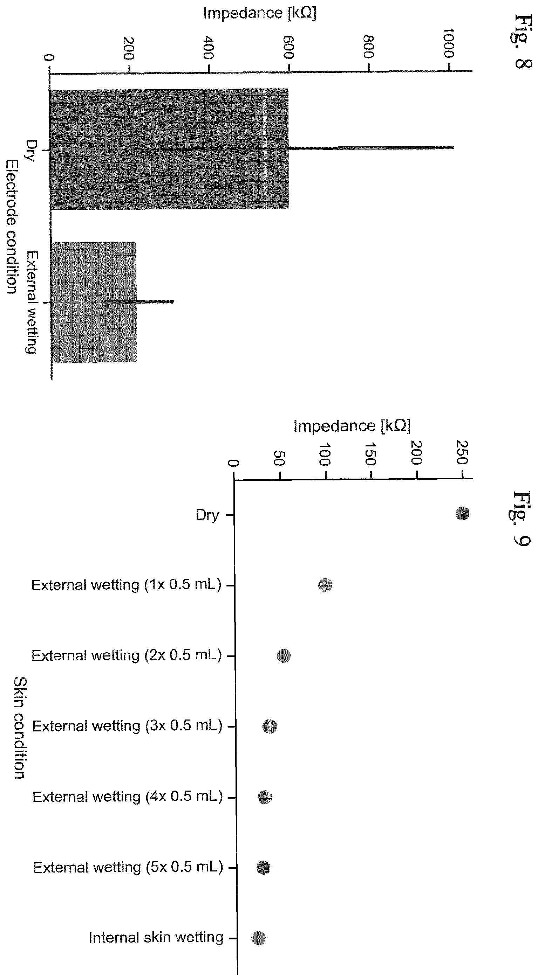

Seven different subjects were asked to wear the T-Shirt. Impedance between the skin of each subject and each of the textile electrodes of the T-Shirt was measured at a frequency of 10 Hz under dry conditions after 10 s wear time using a potentiostat capable of electrochemical impedance spectroscopy with a three-electrode setup. Commercial medical wet electrodes were placed on the forearm as counter and reference electrodes with the textile electrode as the working electrode. An average impedance of 600 kΩ was determined (see Figure 8).

Subsequently, the T-Shirt was wetted from the outside by applying water by hand onto the T-Shirt. Impedance between the skin of each subject and each of the textile electrodes of the T-Shirt was again measured at a frequency of 10 Hz under wetted conditions after 10 s. An average impedance of 200 kΩ was determined (see Figure 8).

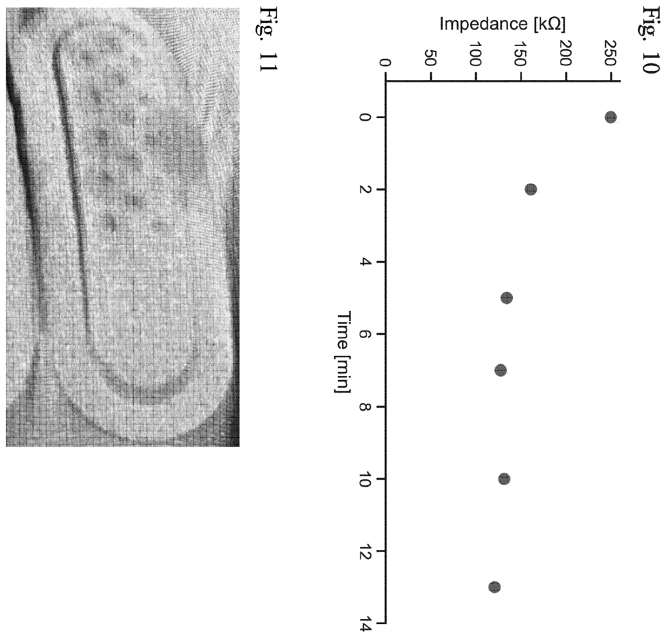

In an additional experiment, the T-shirt was worn by a subject. One electrode was repetitively wetted from the outside by applying 0.5 mL of water with a Pasteur pipette, each time followed by an immediate impedance measurement. The initial dry impedance of 250 kΩ dropped to 99 kΩ after the first time external wetting, continued to decrease after each wetting repetition and reached an impedance value of 29 kΩ after 5 repeats (see Figure 9). Finally, an impedance measurement was conducted after lifting the shirt, fully wetting the skin underneath and repressing the electrode on the shirt. The impedance value of the completely wet skin was 23 kΩ. As a control measurement, a second electrode in the same T-shirt was measured simultaneously over time without wetting. Sweating of the skin reduced the impedance to a value of 120 kΩ after a waiting time of 13 min (see Figure 10). Wetting the electrode externally with 0.5mL reduced the electrode-skin impedance immediately to a lower level than the control after a settling time of 13 min.

Accordingly, a substantial decrease in impedance could be achieved with the textile electrode of the present invention.

Each subject wearing the T-Shirt was then asked to perform extensive exercising in the form of running over 10 minutes. As may be taken from the photograph shown in Figure 11, sweat generated during exercising under the electrodes could apparently penetrate the through holes and wet the outer side of the T-Shirt.

## Claims

1. A textile electrode (1) comprising:
a backing (2) comprising a water-permeable textile material; and
a laminar electrode (3) with first and second surfaces, the first surface being arranged to be in direct contact with skin of a person and the second surface being attached to the backing;
wherein the electrode is electrically conductive and comprises through holes (4) extending from the first surface to the second surface of the electrode, wherein the electrode comprises a material which, at areas without any through holes, has a higher resistance to water penetration than the textile material of the backing;
wherein the electrode material, at areas without any through holes, has a resistance to water penetration that is greater than 10 mBar.

2. The textile electrode according to any one of the preceding claims,
wherein the volume of water that penetrates through the backing within 10 minutes amounts preferably to at least 100 mL, more preferably to at least 250 mL, most preferably to at least 500 mL when exposed to water drops that amount to 800 mL of water in total.

3. The textile electrode according to any one of the preceding claims,
wherein the cross-sectional area of each through hole is greater than 0.4 mm², preferably greater than 1 mm², most preferably greater than 2 mm² and/or wherein the cross-sectional area of each through hole is smaller than 50 mm², preferably smaller than 30 mm², most preferably smaller than 10 mm².

4. The textile electrode according to any one of the preceding claims,
wherein the total cross-sectional area of all through holes amounts to at least 3%, preferably at least 5%, most preferably at least 7% of the area of the first surface of the electrode and/or wherein the total cross-sectional area of all through holes amounts to at most 30%, preferably at most 20%, most preferably at most 10% of the area of the first surface of the electrode.

5. The textile electrode according to any one of the preceding claims,
wherein the cross-section of the through holes has a maximum dimension, wherein the laminar electrode has a thickness and wherein the ratio of the maximum dimension to the thickness is greater than 1, preferably greater than 2, more preferably greater than 3, most preferably greater than 4.

6. The textile electrode according to any one of the preceding claims,
wherein the electrode material, at areas without any through holes, has a water vapor transmission rate lower than 10,000 g/m²/d, preferably lower than 5,000 g/m²/d, most preferably lower than 2,000 g/m²/d.

7. The textile electrode according to any one of the preceding claims,
wherein the first surface of the laminar electrode has a higher water contact angle than the outer surface of the textile backing.

8. The textile electrode according to any one of the preceding claims,
wherein the laminar electrode is attached to the backing by means of an adhesive layer,
wherein the adhesive layer comprises perforations.

9. The textile electrode according to any one of the preceding claims,
wherein the electrode comprises a non-conducting ring.

10. The textile electrode according to any one of the preceding claims,
wherein the electrode further comprises an electrical connector, wherein one end of the electrical connector is sandwiched between the backing and the laminar electrode so as to establish an electrical connection between the laminar electrode and the electrical connector,
wherein the laminar electrode is preferably attached to the backing by means of an adhesive layer and wherein the one end of the electrical connector is preferably sandwiched between the backing and the adhesive layer of the laminar electrode,
wherein the adhesive layer comprises perforations in an area sandwiched between the laminar electrode and the one end of the electrical connector so as to establish an electrical connection between the laminar electrode and the electrical connector.

11. A textile product comprising the textile electrode according to any one of the preceding claims,
wherein the textile material of the backing of the electrode preferably is an integral part of the textile of the textile product.

12. A method of manufacturing the textile electrode according to any one of claims 1 to 10, the method comprising:
providing an electrically conductive laminar electrode with first and second surfaces, the first surface being arranged to be in direct contact with skin of a person, wherein the electrode material has a resistance to water penetration that is greater than 10 mBar;
generating through holes in the laminar electrode extending from the first surface to the second surface of the laminar electrode;
providing a textile backing, wherein the laminar electrode has a higher resistance to water penetration than the textile backing; and
attaching the second surface of the laminar electrode to the textile backing.

13. The method according to claim 12,
wherein the textile backing is water-permeable, wherein the volume of water that penetrates through the textile backing within 10 minutes amounts preferably to at least 100 mL, more preferably to at least 250 mL, most preferably to at least 500 mL when exposed to water drops that amount to 800 mL of water in total.

14. The method according to claim 12 or claim 13,
wherein the laminar electrode has a water vapor transmission rate lower than 10,000 g/m²/d, preferably lower than 5,000 g/m²/d, most preferably lower than 2,000 g/m²/d.

15. The method according to any one of claims 11 to 14,
wherein the step of providing an electrically conductive laminar electrode comprises:
providing a perforated adhesive layer;
bonding the perforated adhesive layer to a conductive fabric; and
coating the conductive fabric with a, preferably adhesive, conductive layer.

## Patentansprüche

1. Textilelektrode (1), die aufweist:
einen Träger (2), der ein wasserdurchlässiges Textilmaterial aufweist; und
eine laminare Elektrode (3) mit einer ersten und einer zweiten Oberfläche, wobei die erste Oberfläche angeordnet ist, um in direktem Kontakt mit der Haut einer Person zu stehen, und die zweite Oberfläche am Träger befestigt ist;
wobei die Elektrode elektrisch leitfähig ist und Durchgangslöcher (4) aufweist, die sich von der ersten Oberfläche zur zweiten Oberfläche der Elektrode erstrecken, wobei die Elektrode ein Material aufweist, das an Bereichen ohne Durchgangslöcher eine höhere Beständigkeit gegen das Eindringen von Wasser aufweist als das Textilmaterial des Trägers;
wobei das Elektrodenmaterial an Bereichen ohne Durchgangslöcher eine Beständigkeit gegen das Eindringen von Wasser aufweist, die größer als 10 mBar ist.

2. Textilelektrode nach einem der vorhergehenden Ansprüche,
wobei das Wasservolumen, das innerhalb von 10 Minuten durch den Träger dringt, vorzugsweise mindestens 100 ml, noch bevorzugter mindestens 250 ml und am meisten bevorzugt mindestens 500 ml beträgt, wenn er Wassertropfen ausgesetzt wird, die insgesamt 800 ml Wasser ausmachen.

3. Textilelektrode nach einem der vorhergehenden Ansprüche,
wobei die Querschnittsfläche jedes Durchgangslochs größer als 0,4 mm² ist, vorzugsweise größer als 1 mm², am bevorzugtesten größer als 2 mm², und/oder wobei die Querschnittsfläche jedes Durchgangslochs kleiner als 50 mm² ist, vorzugsweise kleiner als 30 mm², am bevorzugtesten kleiner als 10 mm².

4. Textilelektrode nach einem der vorhergehenden Ansprüche,
wobei die Gesamtquerschnittsfläche aller Durchgangslöcher mindestens 3 %, vorzugsweise mindestens 5 %, am bevorzugtesten mindestens 7 % der Fläche der ersten Oberfläche der Elektrode beträgt und/oder wobei die Gesamtquerschnittsfläche aller Durchgangslöcher höchstens 30 %, vorzugsweise höchstens 20 %, am bevorzugtesten höchstens 10 % der Fläche der ersten Oberfläche der Elektrode beträgt.

5. Textilelektrode nach einem der vorhergehenden Ansprüche,
wobei der Querschnitt der Durchgangslöcher eine maximale Abmessung aufweist, wobei die laminare Elektrode eine Dicke aufweist und wobei das Verhältnis der maximalen Abmessung zur Dicke größer als 1, vorzugsweise größer als 2, noch bevorzugter größer als 3, am bevorzugtesten größer als 4 ist.

6. Textilelektrode nach einem der vorhergehenden Ansprüche,
wobei das Elektrodenmaterial an Bereichen ohne Durchgangslöcher eine Wasserdampfdurchlassrate von weniger als 10000 g/m²/d, vorzugsweise weniger als 5000 g/m²/d, am bevorzugtesten weniger als 2000 g/m²/d aufweist.

7. Textilelektrode nach einem der vorhergehenden Ansprüche,
wobei die erste Oberfläche der laminaren Elektrode einen höheren Wasserkontaktwinkel aufweist als die Außenfläche des textilen Trägers.

8. Textilelektrode nach einem der vorhergehenden Ansprüche,
wobei die laminare Elektrode mittels einer Klebeschicht am Träger befestigt ist, wobei die Klebeschicht Perforationen aufweist.

9. Textilelektrode nach einem der vorhergehenden Ansprüche,
wobei die Elektrode einen nichtleitenden Ring aufweist.

10. Textilelektrode nach einem der vorhergehenden Ansprüche,
wobei die Elektrode ferner einen elektrischen Verbinder aufweist, wobei ein Ende des elektrischen Verbinders zwischen dem Träger und der laminaren Elektrode angeordnet ist, um eine elektrische Verbindung zwischen der laminaren Elektrode und dem elektrischen Verbinder herzustellen,
wobei die laminare Elektrode vorzugsweise mittels einer Klebeschicht am Träger befestigt ist und wobei das eine Ende des elektrischen Verbinders vorzugsweise zwischen dem Träger und der Klebeschicht der laminaren Elektrode angeordnet ist,
wobei die Klebeschicht Perforationen in einem Bereich aufweist, der zwischen der laminaren Elektrode und dem einen Ende des elektrischen Verbinders angeordnet ist, um eine elektrische Verbindung zwischen der laminaren Elektrode und dem elektrischen Verbinder herzustellen.

11. Textilprodukt, das die Textilelektrode nach einem der vorhergehenden Ansprüche aufweist,
wobei das Textilmaterial des Trägers der Elektrode vorzugsweise ein integraler Teil des Textils des Textilprodukts ist.

12. Verfahren zur Herstellung der Textilelektrode nach einem der Ansprüche 1 bis 10, wobei das Verfahren aufweist:
Bereitstellen einer elektrisch leitfähigen laminaren Elektrode mit einer ersten und einer zweiten Oberfläche, wobei die erste Oberfläche angeordnet ist, um in direktem Kontakt mit der Haut einer Person zu stehen, wobei das Elektrodenmaterial eine Wasserundurchlässigkeit von mehr als 10 mBar aufweist;
Erzeugen von Durchgangslöchern in der laminaren Elektrode, die sich von der ersten Oberfläche zur zweiten Oberfläche der laminaren Elektrode erstrecken;
Bereitstellen eines textilen Trägers, wobei die laminare Elektrode einen höheren Widerstand gegen das Eindringen von Wasser aufweist als der textile Träger; und
Befestigen der zweiten Oberfläche der laminaren Elektrode am textilen Träger.

13. Verfahren nach Anspruch 12,
wobei der textile Träger wasserdurchlässig ist, wobei das Wasservolumen, das innerhalb von 10 Minuten durch den textilen Träger dringt, vorzugsweise mindestens 100 ml, noch bevorzugter mindestens 250 ml und am meisten bevorzugt mindestens 500 ml beträgt, wenn er Wassertropfen ausgesetzt wird, die insgesamt 800 ml Wasser ausmachen.

14. Verfahren nach Anspruch 12 oder Anspruch 13,
wobei die laminare Elektrode eine Wasserdampfdurchlassrate von weniger als 10000 g/m²/d, vorzugsweise weniger als 5000 g/m²/d, am bevorzugtesten weniger als 2000 g/m²/d aufweist.

15. Verfahren nach einem der Ansprüche 11 bis 14,
wobei der Schritt des Bereitstellens einer elektrisch leitfähigen laminaren Elektrode aufweist:
Bereitstellen einer perforierten Klebeschicht;
Verbinden der perforierten Klebeschicht mit einem leitfähigen Gewebe; und
Beschichten des leitfähigen Gewebes mit einer, vorzugsweise klebenden, leitfähigen Schicht.

## Revendications

1. Électrode textile (1) comprenant :
un support (2) constitué d'un matériau textile perméable à l'eau ; et
une électrode laminaire (3) présentant une première et une deuxième surfaces, la première surface étant prévue de manière à être en contact direct avec la peau d'une personne et la deuxième surface étant fixée au support ;
où l'électrode est électriquement conductrice et comprend des trous traversants (4) s'étendant de la première surface à la deuxième surface de l'électrode, l'électrode comprenant un matériau qui, dans les zones dépourvues de trous traversants, présente une résistance à la pénétration d'eau supérieure à celle du matériau textile du support ;
où le matériau de l'électrode, dans les zones dépourvues de trous traversants, présente une résistance à la pénétration de l'eau supérieure à 10 mBar.

2. Électrode textile selon l'une des revendications précédentes,
où le volume d'eau traversant le support en 10 minutes s'élève avantageusement à au moins 100 ml, préférentiellement à au moins 250 ml, et tout particulièrement à au moins 500 ml lors d'une exposition à des gouttes d'eau dont le total s'élève à 800 ml d'eau.

3. Électrode textile selon l'une des revendications précédentes,
où la section transversale de chaque trou traversant est supérieure à 0,4 mm², avantageusement supérieure à 1 mm², préférentiellement supérieure à 2 mm² et/ou où la section transversale de chaque trou traversant est inférieure à 50 mm², avantageusement inférieure à 30 mm², préférentiellement inférieure à 10 mm².

4. Électrode textile selon l'une des revendications précédentes,
où la section transversale totale de tous les trous traversants représente au moins 3 %, avantageusement au moins 5 %, préférentiellement au moins 7 % de la surface de la première face de l'électrode et/ou où la section transversale totale de tous les trous traversants représente au plus 30 %, avantageusement au plus 20 %, préférentiellement au plus 10 % de la surface de la première face de l'électrode.

5. Électrode textile selon l'une des revendications précédentes,
où la section transversale des trous traversants présente une dimension maximale, où l'électrode laminaire présente une épaisseur et où le rapport entre la dimension maximale et l'épaisseur est supérieur à 1, avantageusement supérieur à 2, préférentiellement supérieur à 3, et tout particulièrement supérieur à 4.

6. Électrode textile selon l'une des revendications précédentes,
où le matériau de l'électrode, dans les zones dépourvues de trous traversants, présente un taux de transmission de vapeur d'eau inférieur à 10 000 g/m²/jour, avantageusement inférieur à 5 000 g/m²/jour, préférentiellement inférieur à 2 000 g/m²/jour.

7. Électrode textile selon l'une des revendications précédentes,
où la première surface de l'électrode laminaire présente un angle de contact avec l'eau supérieur à celui de la surface extérieure du support textile.

8. Électrode textile selon l'une des revendications précédentes,
où l'électrode laminaire est fixée au support au moyen d'une couche adhésive, ladite couche adhésive présentant des perforations.

9. Électrode textile selon l'une des revendications précédentes,
où ladite électrode comprend un anneau non conducteur.

10. Électrode textile selon l'une des revendications précédentes,
où ladite électrode comprend en outre un connecteur électrique, une première extrémité du connecteur électrique étant enserrée entre le support et l'électrode laminaire de manière à établir une connexion électrique entre l'électrode laminaire et le connecteur électrique,
où l'électrode laminaire est de préférence fixée au support au moyen d'une couche adhésive et où la première extrémité du connecteur électrique est de préférence enserrée entre le support et la couche adhésive de l'électrode laminaire,
où la couche adhésive présente des perforations dans une zone enserrée entre l'électrode laminaire et la première extrémité du connecteur électrique de manière à établir une connexion électrique entre l'électrode laminaire et le connecteur électrique.

11. Produit textile comprenant l'électrode textile selon l'une des revendications précédentes,
où le matériau textile du support de l'électrode fait de préférence partie intégrante du textile du produit textile.

12. Procédé de fabrication de l'électrode textile selon l'une des revendications 1 à 10, le dit procédé comprenant :
la mise à disposition d'une électrode laminaire électriquement conductrice présentant une première et une deuxième surfaces, la première surface étant prévue de manière à être en contact direct avec la peau d'une personne, le matériau de l'électrode présentant une résistance à la pénétration de l'eau supérieure à 10 mBar ;
la réalisation de trous traversants dans l'électrode laminaire, s'étendant de la première surface à la deuxième surface de l'électrode laminaire ;
la mise à disposition d'un support textile, l'électrode laminaire présentant une résistance à la pénétration d'eau supérieure à celle du support textile ; et
la fixation de la deuxième surface de l'électrode laminaire au support textile.

13. Procédé selon la revendication 12,
où le support textile est perméable à l'eau, le volume d'eau traversant le support en 10 minutes s'élevant avantageusement à au moins 100 ml, préférentiellement à au moins 250 ml, et tout particulièrement à au moins 500 ml lors d'une exposition à des gouttes d'eau dont le total s'élève à 800 ml d'eau.

14. Procédé selon la revendication 12 ou la revendication 13,
où l'électrode laminaire présente un taux de transmission de vapeur d'eau inférieur à 10 000 g/m²/jour, avantageusement inférieur à 5 000 g/m²/jour, préférentiellement inférieur à 2 000 g/m²/jour.

15. Procédé selon l'une des revendications 11 à 14, où l'étape de mise à disposition d'une électrode laminaire électriquement conductrice comprend :
la mise à disposition d'une couche adhésive perforée ;
la liaison de la couche adhésive perforée à un tissu conducteur ; et
le revêtement du tissu conducteur avec une couche conductrice, de préférence adhésive.
